# EUROPEAN PATENT SPECIFICATION

(11) **EP 4 240 473 B1**
(45) Date of publication and mention of the grant of the patent: **29.04.2026**
(21) Application number: 21815814.5
(22) Date of filing: 04.11.2021
(51) Int. Cl.: A61N 1/36

(54) **SYSTEM FOR CONTROLLING THERAPY CONTROL PARAMETER UPDATES**
SYSTEM ZUR STEUERUNG VON THERAPIESTEUERUNGSPARAMETERAKTUALISIERUNGEN
SYSTÈME PERMETTANT DE COMMANDER DES MISES À JOUR DE PARAMÈTRES DE COMMANDE DE THÉRAPIE

(30) Priority: 04.11.2020 US 202063109483 P
(43) Date of publication of application: 13.09.2023
(73) Proprietor: Boston Scientific Neuromodulation Corporation, Valencia, CA 91355 (US)
(72) Inventor: HADDOCK, Andrew James, Los Angeles, California 90027 (US); SERCEKI, Zeljko, John, San Diego, California 92101 (US); JAYAKUMAR, Adarsh, Valencia, California 91355 (US); UYEDA, Joshua, Los Angeles, California 90066 (US)
(74) Representative: Vossius & Partner Patentanwälte Rechtsanwälte mbB
(86) International application number: PCT/US2021/058006
(87) International publication number: WO 2022/098837

(56) References cited:
- US-A1- 2017 056 664
- US-A1- 2018 116 540

## Description

### TECHNICAL FIELD

This document relates generally to medical devices and more particularly to a medical device that modifies parameter update rates in closed-loop control for efficient use of power and computational resources.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device is used to program the implantable neurostimulator with stimulation parameters controlling the delivery of the neurostimulation energy.

In one example, the neurostimulation energy is delivered in a form of electrical signals. The delivery is controlled using stimulation parameters that specify spatial (where to stimulate), temporal (when to stimulate), and informational (patterns of stimulation directing the nervous system to respond as desired) aspects of a pattern of the electrical signals. Efficacy and efficiency of certain neurostimulation therapies can be improved, and their side-effects can be reduced, by determining these stimulation parameters based on a patient's conditions and therapeutic objectives. While modern electronics can accommodate the need for generating sophisticated signal patterns, the capability of a neurostimulation system depends on how stimulation parameters defining such a signal pattern can be determined and adjusted for the patient to ensure efficacy and efficiency of a therapy using neurostimulation when applied to the patient. US2017056664A1 relates to systems, devices, and methods for detecting positional and behavioral states of a patient, and more particularly, to systems, devices, and methods for adapting sensing and therapy delivery based on the detected positional and behavioral states of a patient.

### SUMMARY OF THE INVENTION

The invention is defined in claim 1. Any methods disclosed hereinafter do not form part of the scope of the invention, and are mentioned for illustrative purposes only.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims

### BRIEF DESCRIPTION OF THE DRAWINGS

The drawings illustrate generally, by way of example, various embodiments discussed in the present document. The drawings are for illustrative purposes only and may not be to scale.
FIG. 1 illustrates an embodiment of a neurostimulation system.
FIG. 2 illustrates an embodiment of a stimulation device and a lead system, such as may be implemented in the neurostimulation system of FIG. 1.
FIG. 3 illustrates an embodiment of a programming device, such as may be implemented in the neurostimulation system of FIG. 1.
FIG. 4 illustrates an embodiment of an implantable pulse generator (IPG) and an implantable lead system, such as an example implementation of the stimulation device and lead system of FIG. 2.
FIG. 5 illustrates an embodiment of an IPG and an implantable lead system, such as the IPG and lead system of FIG. 4, arranged to provide neurostimulation to a patient.
FIG. 6 illustrates an embodiment of portions of a neurostimulation system.
FIG. 7 illustrates an embodiment of an implantable stimulator and one or more leads of an implantable neurostimulation system, such as the implantable neurostimulation system of FIG. 6.
FIG. 8 illustrates an embodiment of an external programming device of an implantable neurostimulation system, such as the implantable neurostimulation system of FIG. 6.
FIG. 9 illustrates an embodiment of sensors and a therapy device for delivering a therapy to a patient and controlling the delivery of the therapy using sensed therapy-control signals.
FIG. 10 illustrates an embodiment of a system including a therapy output device and a therapy control circuit, such as implemented in the therapy device of FIG. 9.
FIG. 11 illustrates an embodiment of an implantable medical device that can be figured to be an implantable stimulator, such as the implantable stimulator of FIG. 7.
FIG. 12 illustrates an embodiment of one or more physical state sensors and a physical state detector for detecting a physical state of the patient in a system for therapy control, such as the system of FIG. 10.
FIG. 13 illustrates an embodiment of an update rate adjuster for adjusting one or more parameter update rates in a system for therapy control, such as the system of FIG. 10.
FIG. 14 illustrates an embodiment of a method for delivering a therapy to a patient, such as a method that can be performed by the system of FIG. 10.
FIG. 15 illustrates an example of parameter update rates as a function of rate-adjusting parameters.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized, and that structural, logical and electrical changes may be made without departing from the scope of the present invention. References to "an", "one", or "various" embodiments in this disclosure are not necessarily to the same embodiment, and such references contemplate more than one embodiment. The following detailed description provides examples, and the scope of the present invention is defined by the appended claims

This document discusses, among other things, an implantable medical device that dynamically adjusts update frequency in closed-loop therapy control operations for efficient use of power and computational resources. For example, the implantable medical device can include an implantable pulse generator (IPG) that is battery powered and performs closed-loop operation with on-board sensing and stimulation updates. The rate (i.e., frequency) at which these sensing and stimulation updates are performed is a significant factor determining operational cost of the closed-loop therapy control, including cost of energy and cost of computational resources. An IPG can be battery-powered to be subcutaneously placed in a patient for chronic use. Increased longevity and/or recharging interval of the battery are desirable, and hence battery power conservation is a design objective for the benefit of the patient. Closed-loop sensing and stimulation systems are used in IPGs, including implantable neurostimulators, to provide therapy control that responds to the patient's changing needs and/or conditions, which can be sensed by one or more sensors in the IPG, implanted in the patient and communicatively coupled to the IPG, and/or externally carried by or placed near the patient and communicatively coupled to the IPG. The operation of such a closed-loop system requires energy and adds burden to the computational resources available from the IPG. Such requirements increase with the rate at which the sensing and stimulation updates are performed. A high rate of the sensing and stimulation updates allows a therapy can be adjusted rapidly in response to the patient's changing needs and/or conditions, but consumers more energy and more computational resources (which also means more energy). Thus, there is a need for a rate of the sensing and stimulation updates that allows for an adequate performance of the closed-loop therapy control while minimizing the energy consumption required for the operation of closed-loop therapy control.

The present subject matter controls the rate at which the sensing and stimulation updates are performed in a closed-loop therapy control system based on a detected indicator of how quickly one or more input signal change. A system for controlling the sensing and stimulation uprate rate can be implemented in an IPG, such as an implantable neurostimulator. The therapy update rate can be adjusted based on one or more sensed signals from which the indicator can be detected. The indicator can include the patient's activity level, which can be detected from any one or more signals indicative of the patient's activities that can be sensed using implantable and/or external sensors. The therapy update rate can be increased when necessary as indicated by a high patient activity level and decreased when the patient activity level is low. Other factors can also be used to adjust the therapy update rate. For example, the therapy update rate when the battery level is low, the therapy update rate can be decreased to maintain operation of the IPG at a power-conservation mode.

In an example embodiment, a system for controlling the sensing and stimulation therapy update rate is implemented as firmware in a processor of the IPG that controls the operation of the IPG at a high level, while a co-processor of the IPG provides the main processor with the detected indicator of how quickly the one or more input signal to the closed-loop therapy control system change. Thus, the main processor of an IPG can control the therapy update rate for a closed-loop therapy control system, while the co-processor can detect the patient activity level using signals from one or more sensors. The main processor can track power (battery) resources and computation resources available in the IPG, receive data indicative of the patient activity level from the co-processor, and adjust, for example at certain intervals, the therapy update rate based on the patient activity level.

Determination of the sensing and stimulation therapy update rate can be an optimization process that balances between the risk of inadequate (e.g., insufficient or excessive) stimulation and the amount of power consumption and computation resources (e.g., from the main processor and the co-processor) associated with the therapy update rate. The frequency of execution of a closed-loop therapy control algorithm, measurements of the one or more input signals, and adjustments of the therapy (e.g., stimulation) can be changed on a per measurement basis. In various embodiments, the sensing and stimulation therapy update rate can be the rate (frequency) at which the one or more input signals are measured, or a function of that rate.

While an IPG for neurostimulation is discussed as an example, the present subject matter can be applied to any closed-loop therapy control system, for example to reduce power consumption and/or demand for computational resources. In various embodiments, the present subject matter provides for flexibility of therapy adjustment based on one or more indicators of an ongoing or anticipated need for a certain frequency of the adjustment, such as the patient's activity level and the battery status of the IPG. This can provide for a substantial amount of power and/or computational resource conservation when compared to existing devices that use the constant sensing and stimulation therapy update rate that is determined to accommodate, for example, the highest patient activity level.

In this document, a "patient" includes a person receiving treatment delivered using a therapy system including an IPG, such as a neurostimulation system including an implantable neurostimulator, according to the present subject matter, and a "user" includes a physician or other caregiver who treats the patient using the therapy system.

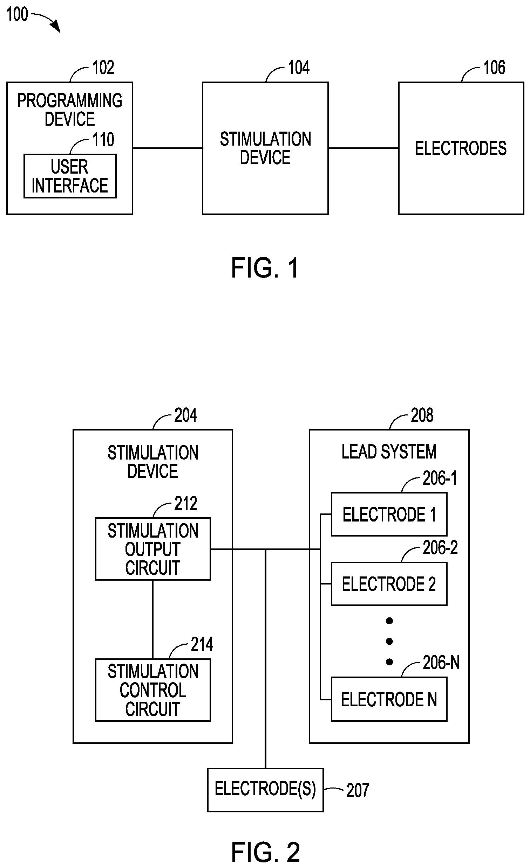

FIG. 1 illustrates an embodiment of a neurostimulation system 100. System 100 includes electrodes 106, a stimulation device 104, and a programming device 102. Electrodes 106 are configured to be placed on or near one or more neural targets in a patient. Stimulation device 104 is configured to be electrically connected to electrodes 106 and deliver neurostimulation energy, such as in the form of electrical pulses, to the one or more neural targets though electrodes 106. The delivery of the neurostimulation is controlled by using a plurality of stimulation parameters, such as stimulation parameters specifying a pattern of the electrical pulses and a selection of electrodes through which each of the electrical pulses is delivered. In various embodiments, at least some parameters of the plurality of stimulation parameters are programmable by a user, such as a physician or other caregiver who treats the patient using system 100. Programming device 102 provides the user with accessibility to the user-programmable parameters. In various embodiments, programming device 102 is configured to be communicatively coupled to stimulation device via a wired or wireless link.

In this document, a "user" includes a physician or other clinician or caregiver who treats the patient using system 100; a "patient" includes a person who receives or is intended to receive neurostimulation delivered using system 100. In various embodiments, the patient can be allowed to adjust his or her treatment using system 100 to certain extent, such as by adjusting certain therapy parameters and entering feedback and clinical effect information.

In various embodiments, programming device 102 can include a user interface 110 that allows the user to control the operation of system 100 and monitor the performance of system 100 as well as conditions of the patient including responses to the delivery of the neurostimulation. The user can control the operation of system 100 by setting and/or adjusting values of the user-programmable parameters.

In various embodiments, user interface 110 can include a graphical user interface (GUI) that allows the user to set and/or adjust the values of the user-programmable parameters by creating and/or editing graphical representations of various waveforms. Such waveforms may include, for example, a waveform representing a pattern of neurostimulation pulses to be delivered to the patient as well as individual waveforms that are used as building blocks of the pattern of neurostimulation pulses, such as the waveform of each pulse in the pattern of neurostimulation pulses. The GUI may also allow the user to set and/or adjust stimulation fields each defined by a set of electrodes through which one or more neurostimulation pulses represented by a waveform are delivered to the patient. The stimulation fields may each be further defined by the distribution of the current of each neurostimulation pulse in the waveform. In various embodiments, neurostimulation pulses for a stimulation period (such as the duration of a therapy session) may be delivered to multiple stimulation fields.

In various embodiments, system 100 can be configured for neurostimulation applications. User interface 110 can be configured to allow the user to control the operation of system 100 for neurostimulation. For example, system 100 as well as user interface 100 can be configured for DBS applications. Such DBS configuration includes various features that may simplify the task of the user in programming stimulation device 104 for delivering DBS to the patient, such as the features discussed in this document.

FIG. 2 illustrates an embodiment of a stimulation device 204 and a lead system 208, such as may be implemented in neurostimulation system 100. Stimulation device 204 represents an example of stimulation device 104 and includes a stimulation output circuit 212 and a stimulation control circuit 214. Stimulation output circuit 212 produces and delivers neurostimulation pulses. Stimulation control circuit 214 controls the delivery of the neurostimulation pulses from stimulation output circuit 212 using the plurality of stimulation parameters, which specifies a pattern of the neurostimulation pulses. Lead system 208 includes one or more leads each configured to be electrically connected to stimulation device 204 and a plurality of electrodes 206 distributed in the one or more leads. The plurality of electrodes 206 includes electrode 206-1, electrode 206-2, ... electrode 206-N, each a single electrically conductive contact providing for an electrical interface between stimulation output circuit 212 and tissue of the patient, where N ≥ 2. The neurostimulation pulses are each delivered from stimulation output circuit 212 through a set of electrodes selected from electrodes 206. In various embodiments, the neurostimulation pulses may include one or more individually defined pulses, and the set of electrodes may be individually definable by the user for each of the individually defined pulses or each of collections of pulse intended to be delivered using the same combination of electrodes. In various embodiments, one or more additional electrodes 207 (each of which may be referred to as a reference electrode) can be electrically connected to stimulation device 204, such as one or more electrodes each being a portion of or otherwise incorporated onto a housing of stimulation device 204. Monopolar stimulation uses a monopolar electrode configuration with one or more electrodes selected from electrodes 206 and at least one electrode from electrode(s) 207. Bipolar stimulation uses a bipolar electrode configuration with two electrodes selected from electrodes 206 and none of electrode(s) 207. Multipolar stimulation uses a multipolar electrode configuration with multiple (two or more) electrodes selected from electrodes 206 and none of electrode(s) 207.

In various embodiments, the number of leads and the number of electrodes on each lead depend on, for example, the distribution of target(s) of the neurostimulation and the need for controlling the distribution of electric field at each target. In one embodiment, lead system 208 includes 2 leads each having 8 electrodes.

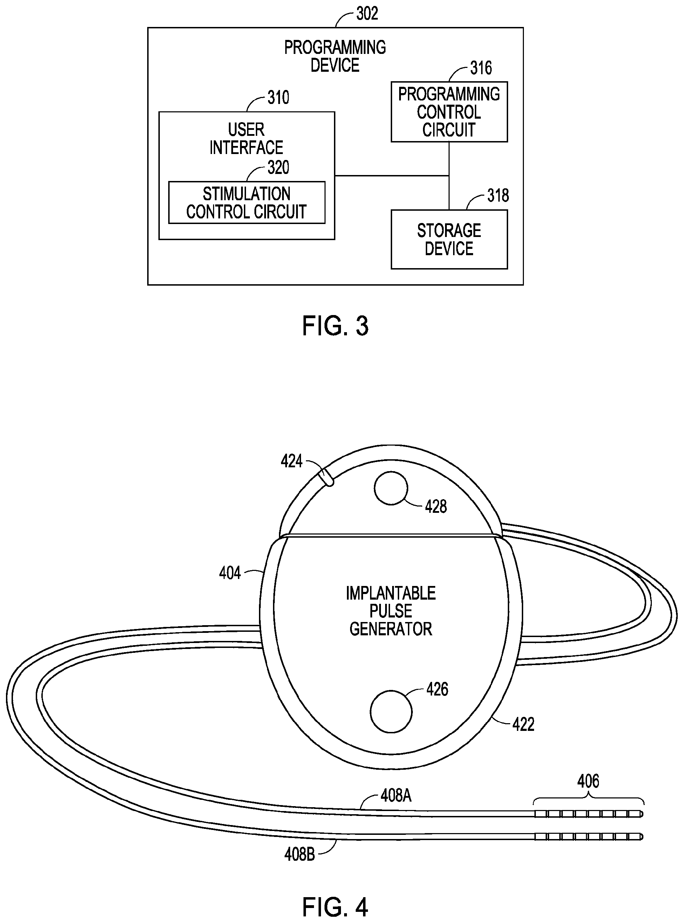

FIG. 3 illustrates an embodiment of a programming device 302, such as may be implemented in neurostimulation system 100. Programming device 302 represents an example of programming device 102 and includes a storage device 318, a programming control circuit 316, and a user interface 310. Programming control circuit 316 generates the plurality of stimulation parameters that controls the delivery of the neurostimulation pulses according to a specified neurostimulation program that can define, for example, stimulation waveform and electrode configuration. User interface 310 represents an example of user interface 110 and includes a stimulation control circuit 320. Storage device 318 stores information used by programming control circuit 316 and stimulation control circuit 320, such as information about a stimulation device that relates the neurostimulation program to the plurality of stimulation parameters. In various embodiments, stimulation control circuit 320 can be configured to support one or more functions allowing for programming of stimulation devices, such as stimulation device 104 including its various embodiments as discussed in this document, according to one or more selected neurostimulation programs as discussed in this document.

In various embodiments, user interface 310 can allow for definition of a pattern of neurostimulation pulses for delivery during a neurostimulation therapy session by creating and/or adjusting one or more stimulation waveforms using a graphical method. The definition can also include definition of one or more stimulation fields each associated with one or more pulses in the pattern of neurostimulation pulses. As used in this document, a "neurostimulation program" can include the pattern of neurostimulation pulses including the one or more stimulation fields, or at least various aspects or parameters of the pattern of neurostimulation pulses including the one or more stimulation fields. In various embodiments, user interface 310 includes a GUI that allows the user to define the pattern of neurostimulation pulses and perform other functions using graphical methods. In this document, "neurostimulation programming" can include the definition of the one or more stimulation waveforms, including the definition of one or more stimulation fields.

In various embodiments, circuits of neurostimulation 100, including its various embodiments discussed in this document, may be implemented using a combination of hardware and software. For example, the circuit of user interface 110, stimulation control circuit 214, programming control circuit 316, and stimulation control circuit 320, including their various embodiments discussed in this document, may be implemented using an application-specific circuit constructed to perform one or more particular functions or a general-purpose circuit programmed to perform such function(s). Such a general-purpose circuit includes, but is not limited to, a microprocessor or a portion thereof, a microcontroller or portions thereof, and a programmable logic circuit or a portion thereof.

FIG. 4 illustrates an embodiment of an implantable pulse generator (IPG) 404 and an implantable lead system 408. IPG 404 represents an example implementation of stimulation device 204. Lead system 408 represents an example implementation of lead system 208. As illustrated in FIG. 4, IPG 404 that can be coupled to implantable leads 408A and 408B at a proximal end of each lead. The distal end of each lead includes electrical contacts or electrodes 406 for contacting a tissue site targeted for electrical neurostimulation. As illustrated in FIG. 1, leads 408A and 408B each include 8 electrodes 406 at the distal end. The number and arrangement of leads 408A and 408B and electrodes 406 as shown in FIG. 1 are only an example, and other numbers and arrangements are possible. In various embodiments, the electrodes are ring electrodes. The implantable leads and electrodes may be configured by shape and size to provide electrical neurostimulation energy to a neuronal target included in the subject's brain or configured to provide electrical neurostimulation energy to a nerve cell target included in the subject's spinal cord.

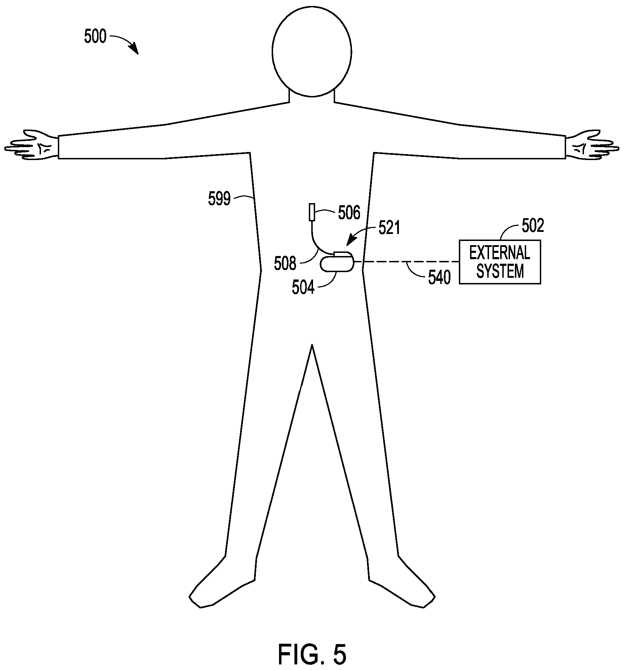

FIG. 5 illustrates an implantable neurostimulation system 500 and portions of an environment in which system 500 may be used. System 500 includes an implantable system 521, an external system 502, and a telemetry link 540 providing for wireless communication between implantable system 521 and external system 502. Implantable system 521 is illustrated in FIG. 5 as being implanted in the patient's body 599.

Implantable system 521 includes an implantable stimulator (also referred to as an implantable pulse generator, or IPG) 504, a lead system 508, and electrodes 506, which represent an example of stimulation device 204, lead system 208, and electrodes 206, respectively. External system 502 represents an example of programming device 302. In various embodiments, external system 502 includes one or more external (non-implantable) devices each allowing the user and/or the patient to communicate with implantable system 521. In some embodiments, external 502 includes a programming device intended for the user to initialize and adjust settings for implantable stimulator 504 and a remote control device intended for use by the patient. For example, the remote control device may allow the patient to turn implantable stimulator 504 on and off and/or adjust certain patient-programmable parameters of the plurality of stimulation parameters.

The sizes and sharps of the elements of implantable system 521 and their location in body 599 are illustrated by way of example and not by way of restriction. An implantable system is discussed as a specific application of the programming according to various embodiments of the present subject matter. In various embodiments, the present subject matter may be applied in programming any type of stimulation device that uses electrical pulses as stimuli, regarding less of stimulation targets in the patient's body and whether the stimulation device is implantable.

Returning to FIG. 4, the IPG 404 can include a hermetically sealed IPG case 422 to house the electronic circuitry of IPG 404. IPG 404 can include an electrode 426 formed on IPG case 422. IPG 404 can include an IPG header 424 for coupling the proximal ends of leads 408A and 408B. IPG header 424 may optionally also include an electrode 428. Electrodes 426 and/or 428 represent embodiments of electrode(s) 207 and may each be referred to as a reference electrode. Neurostimulation energy can be delivered in a monopolar (also referred to as unipolar) mode using electrode 426 or electrode 428 and one or more electrodes selected from electrodes 406. Neurostimulation energy can be delivered in a bipolar mode using a pair of electrodes of the same lead (lead 408A or lead 408B). Neurostimulation energy can be delivered in an extended bipolar mode using one or more electrodes of a lead (e.g., one or more electrodes of lead 408A) and one or more electrodes of a different lead (e.g., one or more electrodes of lead 408B).

The electronic circuitry of IPG 404 can include a control circuit that controls delivery of the neurostimulation energy. The control circuit can include a microprocessor, a digital signal processor, application specific integrated circuit (ASIC), or other type of processor, interpreting or executing instructions included in software or firmware. The neurostimulation energy can be delivered according to specified (e.g., programmed) modulation parameters. Examples of setting modulation parameters can include, among other things, selecting the electrodes or electrode combinations used in the stimulation, configuring an electrode or electrodes as the anode or the cathode for the stimulation, specifying the percentage of the neurostimulation provided by an electrode or electrode combination, and specifying stimulation pulse parameters. Examples of pulse parameters include, among other things, the amplitude of a pulse (specified in current or voltage), pulse duration (e.g., in microseconds), pulse rate (e.g., in pulses per second), and parameters associated with a pulse train or pattern such as burst rate (e.g., an "on" modulation time followed by an "off" modulation time), amplitudes of pulses in the pulse train, polarity of the pulses, etc.

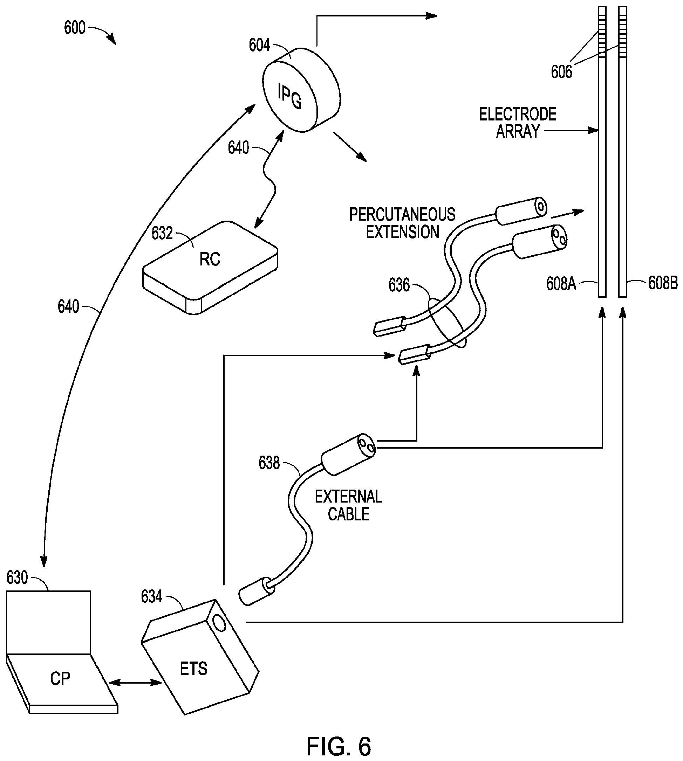

FIG. 6 illustrates an embodiment of portions of a neurostimulation system 600. System 600 includes an IPG 604, implantable neurostimulation leads 608A and 608B, an external remote controller (RC) 632, a clinician's programmer (CP) 630, and an external trial stimulator (ETS, also referred to as external trial modulator, or ETM) 634. IPG 404 may be electrically coupled to leads 608A and 608B directly or through percutaneous extension leads 636. ETS 634 may be electrically connectable to leads 608A and 608B via one or both of percutaneous extension leads 636 and/or external cable 638. System 600 represents an example of system 100, with IPG 604 representing an embodiment of stimulation device 104, electrodes 606 of leads 608A and 608B representing electrodes 106, and CP 630, RC 632, and ETS 634 collectively representing programming device 102.

ETS 634 may be standalone or incorporated into CP 630. ETS 634 may have similar pulse generation circuitry as IPG 604 to deliver neurostimulation energy according to specified modulation parameters as discussed above. ETS 634 is an external device that is typically used as a preliminary stimulator after leads 408A and 408B have been implanted and used prior to stimulation with IPG 604 to test the patient's responsiveness to the stimulation that is to be provided by IPG 604. Because ETS 634 is external it may be more easily configurable than IPG 604.

CP 630 can configure the neurostimulation provided by ETS 634. If ETS 634 is not integrated into CP 630, CP 630 may communicate with ETS 634 using a wired connection (e.g., over a USB link) or by wireless telemetry using a wireless communications link 640. CP 630 also communicates with IPG 604 using a wireless communications link 640.

An example of wireless telemetry is based on inductive coupling between two closely placed coils using the mutual inductance between these coils. This type of telemetry is referred to as inductive telemetry or near-field telemetry because the coils must typically be closely situated for obtaining inductively coupled communication. IPG 604 can include the first coil and a communication circuit. CP 630 can include or otherwise electrically connected to the second coil such as in the form of a wand that can be place near IPG 604. Another example of wireless telemetry includes a far-field telemetry link, also referred to as a radio frequency (RF) telemetry link. A far-field, also referred to as the Fraunhofer zone, refers to the zone in which a component of an electromagnetic field produced by the transmitting electromagnetic radiation source decays substantially proportionally to 1/r, where r is the distance between an observation point and the radiation source. Accordingly, far-field refers to the zone outside the boundary of r = λ/2π, where λ is the wavelength of the transmitted electromagnetic energy. In one example, a communication range of an RF telemetry link is at least six feet but can be as long as allowed by the particular communication technology. RF antennas can be included, for example, in the header of IPG 604 and in the housing of CP 630, eliminating the need for a wand or other means of inductive coupling. An example is such an RF telemetry link is a Bluetooth^{®} wireless link.

CP 630 can be used to set modulation parameters for the neurostimulation after IPG 604 has been implanted. This allows the neurostimulation to be tuned if the requirements for the neurostimulation change after implantation. CP 630 can also upload information from IPG 604.

RC 632 also communicates with IPG 604 using a wireless link 340. RC 632 may be a communication device used by the user or given to the patient. RC 632 may have reduced programming capability compared to CP 630. This allows the user or patient to alter the neurostimulation therapy but does not allow the patient full control over the therapy. For example, the patient may be able to increase the amplitude of neurostimulation pulses or change the time that a preprogrammed stimulation pulse train is applied. RC 632 may be programmed by CP 630. CP 630 may communicate with the RC 632 using a wired or wireless communications link. In some embodiments, CP 630 can program RC 632 when remotely located from RC 632.

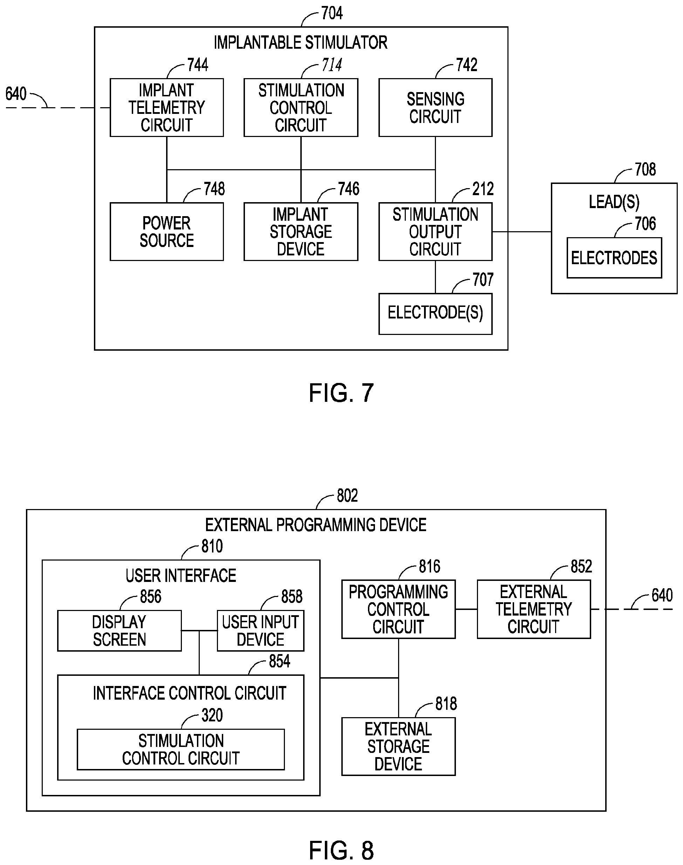

FIG. 7 illustrates an embodiment of implantable stimulator 704 and one or more leads 708 of an implantable neurostimulation system, such as implantable system 600. Implantable stimulator 704 represents an example of stimulation device 104 or 204 and may be implemented, for example, as IPG 604. Lead(s) 708 represents an example of lead system 208 and may be implemented, for example, as implantable leads 608A and 608B. Lead(s) 708 includes electrodes 706, which represents an example of electrodes 106 or 206 and may be implemented as electrodes 606.

Implantable stimulator 704 may include a sensing circuit 742 that is optional and required only when the stimulator needs a sensing capability, stimulation output circuit 212, a stimulation control circuit 714, an implant storage device 746, an implant telemetry circuit 744, a power source 748, and one or more electrodes 707. Sensing circuit 742, when included and needed, senses one or more physiological signals for purposes of patient monitoring and/or feedback control of the neurostimulation. Examples of the one or more physiological signals include neural and other signals each indicative of a condition of the patient that is treated by the neurostimulation and/or a response of the patient to the delivery of the neurostimulation. Stimulation output circuit 212 is electrically connected to electrodes 706 through one or more leads 708 as well as electrodes 707 and delivers each of the neurostimulation pulses through a set of electrodes selected from electrodes 706 and electrode(s) 707. Stimulation control circuit 714 represents an example of stimulation control circuit 214 and controls the delivery of the neurostimulation pulses using the plurality of stimulation parameters specifying the pattern of neurostimulation pulses. In one embodiment, stimulation control circuit 714 controls the delivery of the neurostimulation pulses using the one or more sensed physiological signals. Implant telemetry circuit 744 provides implantable stimulator 704 with wireless communication with another device such as CP 630 and RC 632, including receiving values of the plurality of stimulation parameters from the other device. Implant storage device 746 can store one or more neurostimulation programs and values of the plurality of stimulation parameters for each of the one or more neurostimulation programs. Power source 748 provides implantable stimulator 704 with energy for its operation. In one embodiment, power source 748 includes a battery. In one embodiment, power source 748 includes a rechargeable battery and a battery charging circuit for charging the rechargeable battery. Implant telemetry circuit 744 may also function as a power receiver that receives power transmitted from an external device through an inductive couple. Electrode(s) 707 allow for delivery of the neurostimulation pulses in the monopolar mode. Examples of electrode(s) 707 include electrode 426 and electrode 418 in IPG 404 as illustrated in FIG. 4.

In one embodiment, implantable stimulator 704 is used as a master database. A patient implanted with implantable stimulator 704 (such as may be implemented as IPG 604) may therefore carry patient information needed for his or her medical care when such information is otherwise unavailable. Implant storage device 746 is configured to store such patient information. For example, the patient may be given a new RC 632 and/or travel to a new clinic where a new CP 630 is used to communicate with the device implanted in him or her. The new RC 632 and/or CP 630 can communicate with implantable stimulator 704 to retrieve the patient information stored in implant storage device 746 through implant telemetry circuit 744 and wireless communication link 640 and allow for any necessary adjustment of the operation of implantable stimulator 704 based on the retrieved patient information. In various embodiments, the patient information to be stored in implant storage device 746 may include, for example, positions of lead(s) 708 and electrodes 706 relative to the patient's anatomy (transformation for fusing computerized tomogram (CT) of post-operative lead placement to magnetic resonance imaging (MRI) of the brain), clinical effect map data, objective measurements using quantitative assessments of symptoms (for example using micro-electrode recording, accelerometers, and/or other sensors), and/or any other information considered important or useful for providing adequate care for the patient. In various embodiments, the patient information to be stored in implant storage device 746 may include data transmitted to implantable stimulator 704 for storage as part of the patient information and data acquired by implantable stimulator 704, such as by using sensing circuit 742.

In various embodiments, sensing circuit 742 (if included), stimulation output circuit 212, stimulation control circuit 714, implant telemetry circuit 744, implant storage device 746, and power source 748 are encapsulated in a hermetically sealed implantable housing or case, and electrode(s) 707 are formed or otherwise incorporated onto the case. In various embodiments, lead(s) 708 are implanted such that electrodes 706 are placed on and/or around one or more targets to which the neurostimulation pulses are to be delivered, while implantable stimulator 704 is subcutaneously implanted and connected to lead(s) 708 at the time of implantation.

FIG. 8 illustrates an embodiment of an external programming device 802 of an implantable neurostimulation system, such as system 600. External programming device 802 represents an example of programming device 102 or 302, and may be implemented, for example, as CP 630 and/or RC 632. External programming device 802 includes an external telemetry circuit 852, an external storage device 818, a programming control circuit 816, and a user interface 810.

External telemetry circuit 852 provides external programming device 802 with wireless communication with another device such as implantable stimulator 704 via wireless communication link 640, including transmitting the plurality of stimulation parameters to implantable stimulator 704 and receiving information including the patient data from implantable stimulator 704. In one embodiment, external telemetry circuit 852 also transmits power to implantable stimulator 704 through an inductive couple.

In various embodiments, wireless communication link 640 can include an inductive telemetry link (near-field telemetry link) and/or a far-field telemetry link (RF telemetry link). For example, because DBS is often indicated for movement disorders which are assessed through patient activities, gait, balance, etc., allowing patient mobility during programming and assessment is useful. Therefore, when system 600 is intended for applications including DBS, wireless communication link 640 includes at least a far-field telemetry link that allows for communications between external programming device 802 and implantable stimulator 704 over a relative long distance, such as up to about 20 meters. External telemetry circuit 852 and implant telemetry circuit 744 each include an antenna and RF circuitry configured to support such wireless telemetry.

External storage device 818 stores one or more stimulation waveforms for delivery during a neurostimulation therapy session, such as a DBS therapy session, as well as various parameters and building blocks for defining one or more waveforms. The one or more stimulation waveforms may each be associated with one or more stimulation fields and represent a pattern of neurostimulation pulses to be delivered to the one or more stimulation field during the neurostimulation therapy session. In various embodiments, each of the one or more stimulation waveforms can be selected for modification by the user and/or for use in programming a stimulation device such as implantable stimulator 704 to deliver a therapy. In various embodiments, each waveform in the one or more stimulation waveforms is definable on a pulse-by-pulse basis, and external storage device 818 may include a pulse library that stores one or more individually definable pulse waveforms each defining a pulse type of one or more pulse types. External storage device 818 also stores one or more individually definable stimulation fields. Each waveform in the one or more stimulation waveforms is associated with at least one field of the one or more individually definable stimulation fields. Each field of the one or more individually definable stimulation fields is defined by a set of electrodes through a neurostimulation pulse is delivered. In various embodiments, each field of the one or more individually definable fields is defined by the set of electrodes through which the neurostimulation pulse is delivered and a current distribution of the neurostimulation pulse over the set of electrodes. In one embodiment, the current distribution is defined by assigning a fraction of an overall pulse amplitude to each electrode of the set of electrodes. Such definition of the current distribution may be referred to as "fractionalization" in this document. In another embodiment, the current distribution is defined by assigning an amplitude value to each electrode of the set of electrodes. For example, the set of electrodes may include 2 electrodes used as the anode and an electrode as the cathode for delivering a neurostimulation pulse having a pulse amplitude of 4 mA. The current distribution over the 2 electrodes used as the anode needs to be defined. In one embodiment, a percentage of the pulse amplitude is assigned to each of the 2 electrodes, such as 75% assigned to electrode 1 and 25% to electrode 2. In another embodiment, an amplitude value is assigned to each of the 2 electrodes, such as 3 mA assigned to electrode 1 and 1 mA to electrode 2. Control of the current in terms of percentages allows precise and consistent distribution of the current between electrodes even as the pulse amplitude is adjusted. It is suited for thinking about the problem as steering a stimulation locus, and stimulation changes on multiple contacts simultaneously to move the locus while holding the stimulation amount constant. Control and displaying the total current through each electrode in terms of absolute values (e.g. mA) allows precise dosing of current through each specific electrode. It is suited for changing the current one contact at a time (and allows the user to do so) to shape the stimulation like a piece of clay (pushing/pulling one spot at a time).

Programming control circuit 816 represents an example of programming control circuit 316 and generates the plurality of stimulation parameters, which is to be transmitted to implantable stimulator 704, based on a specified neurostimulation program (e.g., the pattern of neurostimulation pulses as represented by one or more stimulation waveforms and one or more stimulation fields, or at least certain aspects of the pattern). The neurostimulation program may be created and/or adjusted by the user using user interface 810 and stored in external storage device 818. In various embodiments, programming control circuit 816 can check values of the plurality of stimulation parameters against safety rules to limit these values within constraints of the safety rules. In one embodiment, the safety rules are heuristic rules.

User interface 810 represents an example of user interface 310 and allows the user to define the pattern of neurostimulation pulses and perform various other monitoring and programming tasks. User interface 810 includes a display screen 856, a user input device 858, and an interface control circuit 854. Display screen 856 may include any type of interactive or non-interactive screens, and user input device 858 may include any type of user input devices that supports the various functions discussed in this document, such as touchscreen, keyboard, keypad, touchpad, trackball, joystick, and mouse. In one embodiment, user interface 810 includes a GUI. The GUI may also allow the user to perform any functions discussed in this document where graphical presentation and/or editing are suitable as may be appreciated by those skilled in the art.

Interface control circuit 854 controls the operation of user interface 810 including responding to various inputs received by user input device 858 and defining the one or more stimulation waveforms. Interface control circuit 854 includes stimulation control circuit 320.

In various embodiments, external programming device 802 can have operation modes including a composition mode and a real-time programming mode. Under the composition mode (also known as the pulse pattern composition mode), user interface 810 is activated, while programming control circuit 816 is inactivated. Programming control circuit 816 does not dynamically updates values of the plurality of stimulation parameters in response to any change in the one or more stimulation waveforms. Under the real-time programming mode, both user interface 810 and programming control circuit 816 are activated. Programming control circuit 816 dynamically updates values of the plurality of stimulation parameters in response to changes in the set of one or more stimulation waveforms and transmits the plurality of stimulation parameters with the updated values to implantable stimulator 704.

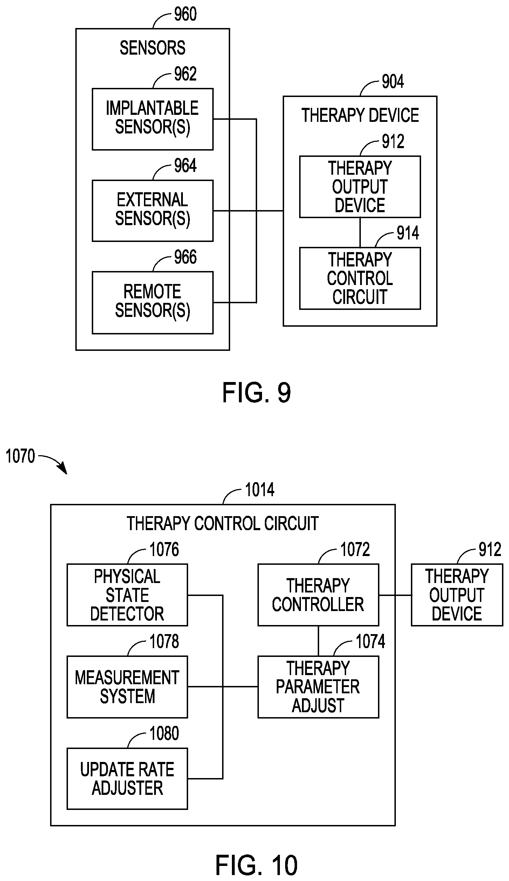

FIG. 9 illustrates an embodiment of sensors 960 and a therapy device 904 for delivering a therapy to a patient and controlling the delivery of the therapy using sensed therapy-control signals. Therapy device 904 can be implemented as stimulation device 104, including but not limited to the various embodiments of stimulation device 104 discussed in this document, for delivering neurostimulation energy and controlling the delivery of the neurostimulation energy using therapy-control signals sensed using sensors 960. The therapy-control signals can include input signals of a closed-loop system that adjusts the delivery of the therapy based on the patient's conditions and/or responses to the therapy. Sensors 960 can include one or more implantable sensors 962 that are configured to be implanted in the patient, one or more external sensors 964 that are configured to be externally attached to or otherwise worn by the patient, and/or one or more remote sensors 966 that are configured to be placed remotely from the patient. When therapy device 904 is configured to be an implantable therapy device, such as implantable stimulator 704, implantable sensor(s) 962 can each be included in the implantable therapy device and/or configured to be a separate implantable device that is communicatively coupled to the implantable therapy device via a wired or wireless connection. External sensor(s) 964 and remote(s) 966 can each be configured to be communicatively coupled to the implantable therapy device via a wireless connection. In one embodiment, sensors 960 include only implantable sensors 962. In other embodiments, sensors 960 can include any combination of implantable sensor(s) 962, external sensor(s) 964, and remote sensors 966.

Sensors 960 can include one or more physiological sensors to sense one or more physiological signals from the patient. The one or more physiological signals can include one or more electrophysiological signals such as one or more neural signals. Examples of such one or more neural signals can include signals indicative of local field potential (LFP, an oscillatory signal from the brain) and/or evoked compound action potential (ECAP). A sensor for sensing such a neural signal can include electrodes, such as electrodes incorporated onto one or more implantable leads (e.g., electrodes 406 on leads 408A and 408B) and/or an implantable medical device (e.g., IPG 404, with electrode 426 on IPG case 422 and/or electrode 428 IPG header 424).

Sensors 960 can include one or more sensors to sense a physical state of the patient. In various embodiments, the physical state can be used as an indicator for how fast the sensed one or more physiological signals are changing and hence, how frequent they should be measured for adjusting the delivery to therapy in a timely manner based on the patient's changing needs as indicated by the sensed one or more physiological signals. In various embodiments, the physical state includes an activity level, and sensors 960 include one or more activity sensors to sense one or more activity signals indicative of the activity level of the patient. The one or more activity sensors can include one or more accelerometers. When therapy device 904 is configured to be an implantable therapy device such as IPG 404, the one or more accelerometers can each be included in IPG 404 or incorporated onto implantable leads 408A or 408B.

Sensors 960 can include one or more sensors to sense one or more environmental signals indicative of an environment of the patient. Examples of the one or more environmental signals include temperature, pressure, humidity, altitude, and other signals that can affect certain physiological functions, such as neural activities, of the patient.

Therapy device 904 can include a therapy output device 912 and a therapy control circuit 914. Therapy output device 912 can deliver the therapy to the patient. Therapy control circuit 914 can control the delivery of the therapy using the therapy-control signals sensed using sensors 960. In various embodiments, therapy control circuit 914 controls the delivery of the therapy by executing a closed-loop control algorithm using one or more sensed input parameters derived from the sensed therapy-control signals. Sensors 906 include sensors that sense the signals used to derive the one or more sensed input parameters.

Therapy device 904 can be configured to be an implantable medical device such as an IPG that delivers the therapy in a form of electrical stimulation pulses to the patient. In one embodiment, therapy device 904 is implemented as an implantable stimulator, such as implantable stimulator 704, to deliver neurostimulation to the patient. The neurostimulation can be delivered, for example, as neurostimulation pulses. Therapy device 912 can be implemented as 212, and therapy control circuit 914 can be implemented as stimulation control circuit 714. This provides stimulation control circuit 714 with closed-loop control of the delivery of the neurostimulation from stimulation output circuit using the sensed therapy-control signals.

FIG. 10 illustrates an embodiment of a system 1070 including therapy output device 912 and a therapy control circuit 1014. Therapy control circuit 1014 can represent an example of therapy device 914 and can receive the sensed therapy-control signals and control the delivery of the therapy using the sensed therapy-control signals. In the illustrated embodiment, therapy control circuit 1014 includes a therapy controller 1072, a therapy parameter adjuster 1074, a physical state detector 1076, a measurement system 1078, and an update rate adjuster 1080.

Therapy controller 1072 can control the delivery of the therapy using therapy parameters. The delivery of the therapy can be controlled by executing a closed-loop control algorithm that uses the therapy parameters. Therapy parameter adjuster 1074 can adjust the therapy parameters using one or more sensed input parameters that are derived from the sensed therapy-control signals. In various embodiments, therapy parameter adjuster 1074 adjusts the therapy parameters using the one or more sensed input parameters at a therapy update rate. The therapy update rate is the frequency at which the therapy parameters are to be adjusted in response to a change in the one or more sensed input parameters. In other words, the therapy update rate is the frequency at which measured value(s) of the one or more sensed input parameters can be used to adjust the stimulation parameters.

Physical state detector 1076 can detect a physical state of the patient using one or more physical signals of the sensed therapy-control signals. Examples of the physical state include sleeping, cooking, sitting, exercising, showering, and walking. Physical states of the patient to be detected can be determined by the need for therapy control and design considerations related to sensing and processing of the sensed signals. In various embodiments, physical state detector 1076 can detect an activity level using the sensed one or more activity signals. The activity level is a measure of a physical activity level of the patient. In one embodiment, the physical state of the patient is represented by the activity level alone. In some other embodiments, the physical state of the patient can be determined by the activity level and one or more sensor signals. Measurement system 1078 can measure one or more signals of the sensed therapy-control signals at a sensing update rate and produce the one or more sensed input parameters based on the measurement. In various embodiments, measurement system 1078 can be configured to perform various functions required for producing the one or more sensed input parameters by measuring the one or more signals of the sensed therapy-control signals. The sensing update rate is the frequency at which the one or more sensed signals are measured to produce the one or more sensed input parameters. In various embodiments, the sensing update rate determines when the next measurement is to be taken, while the therapy update rate determines when the next adjustment of the stimulation parameters is to be performed. Update rate adjuster 1080 can adjust the sensing update rate based on one or more rate-adjusting parameters including the detected physical state of the patient. In various embodiments, update rate adjuster 1080 can adjust the sensing update rate and the therapy update rate based on one or more rate-adjusting parameters including the detected physical state. Other one or more rate-adjusting parameters can include one or more parameters quantitatively indicative of energy and/or computational resource available for use by system 1070.

System 1070 can be implemented in therapy devices, including stimulation devices, discussed in this document (e.g., stimulation device 104, stimulation device 204, IPG 404, IPG 504, IPG 604, implantable stimulator 704, therapy device 904, and implantable medical device 1104). When closed-loop control is used in such a therapy device, the present subject matter prevents the therapy control circuit from constantly using sensing update rate and therapy update rate that are established based on worse-case scenarios that require updates at a high frequency, thereby prolonging battery lifespan and/or recharge cycles and freeing up computation resources that may be used to improve other functions of the implantable medical device and/or save energy used for computation.

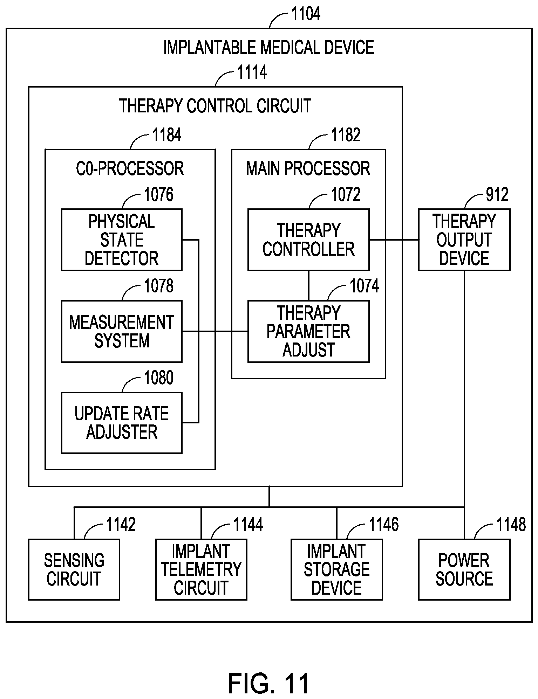

FIG. 11 illustrates an embodiment of an implantable medical device 1104. Implantable medical device 1104 can represent an example of therapy device 904 and can be figured to be an implantable stimulator, such as implantable stimulator 704. In the illustrated embodiment, implantable medical device 1104 includes therapy output device 912, a sensing circuit 1142, an implant telemetry circuit 1144, an implant storage device 1146, a power source 1148, and a therapy control circuit 1114. Implantable medical device 1104 can be figured to be implantable stimulator 704, with therapy output device 912 implemented in stimulation output circuit 212, sensing circuit 1142 implemented in sensing circuit 742, implant telemetry circuit 1144 implemented in implant telemetry circuit 744, implant storage device 1146 implemented in implant storage device 746, power source 1148 implemented in power source 748, and therapy control circuit 1114 implemented in stimulation control circuit 714.

Sensing circuit 1142 can receive the sensed therapy-control signals from sensors such as sensors 960. In various embodiments, sensing circuit 1142 can condition one or more signals of the therapy-control signals in preparation for measurements, such as by amplification and filtering.

Implant telemetry circuit 1144 can provide implantable medical device 1104 with wireless bi-direction communication. In various embodiments, the wireless bi-direction communication can be performed using near-field magnetic telemetry and/or far-field electromagnetic telemetry. In one embodiment, a Bluetooth or Bluetooth Low Energy (BLE) protocol is used for the wireless bi-direction communication.

Implant storage device 1146 can store information required for the operation of implantable medical device 1104 and information acquired by implantable medical device 1104. The information required for the operation of implantable medical device 1104 one or more therapy programs, such as one or more neurostimulation programs, and therapy parameters for each of the one or more therapy programs.

Power source 1148 can include one or more batteries. The amount of energy remaining in the one or more batteries can be indicated by a battery level. The battery level can be measured as a percentage of an energy capacity of the one or more batteries. In various embodiments, the one or more batteries can include rechargeable batteries.

Therapy control circuit 1114 can represent an example of therapy control circuit 1014 being implemented in an implantable medical device. In the illustrated embodiment, therapy control circuit 1114 is implemented in a main processor 1182 and a co-processor 1184. Main processor 1182 includes therapy controller 1072 and therapy parameter adjuster 1074. Co-processor 1184 includes physical state detector 1076, measurement system 1078, and update rate adjuster 1080. In various embodiments, in addition to executing the closed-loop control algorithm as discussed above, main processor 1182 can execute one or more additional sensing-based algorithms using one or more sensed input parameters (e.g., diagnostic algorithms and/or control algorithms for one or more additional therapies). Main processor 1182 can also control the wireless communications to and from implantable medical device 1104 using implant telemetry circuit 1144. Main processor 1182 and co-processor 1184 can include circuits implemented in a single processor or multiple processors. In one embodiment, a microcontroller is configured to be main processor 1182, and another microcontroller is configured to be co-processor 1184. In some embodiments, a microcontroller is configured to be main processor 1182, and additional microcontrollers are configured to be co-processors each performing a function of therapy control circuit 1114.

Update rate adjuster 1080 can adjust at least the sensing update rate based on the one or more rate-adjusting parameters according to a relationship relating the sensing update rate to the one or more rate-adjusting parameters. In various embodiments, update rate adjuster 1080 can adjust the sensing update rate and the therapy update rate according to a relationship relating each of the sensing update rate and the therapy update rate to the one or more rate-adjusting parameters. The one or more rate-adjusting parameters can include the detected physical state (e.g., the activity level) of the patient, a battery state being a measure of the remaining energy in power source 1148, a main processor state being a measure of a level of computational activities in main processor 1182, a co-processor state being a measure of a level of computational activities in co-processor 1184, and/or one or more other parameters indicative of need of implantable medical device 1104 for computational resources and/or energy. In one embodiment, the sensing update rate is equal to the therapy update rate. In another embodiment, the sensing update rate is higher than the therapy update rate (e.g., to allow for multiple measurements of the one or more sensed signals for producing the one or more sensed input parameters by averaging). In another embodiment, the sensing update rate is lower than the therapy update rate (e.g., to allow for both an immediate therapy change and a scheduled therapy change at a later time, before the next sensing update).

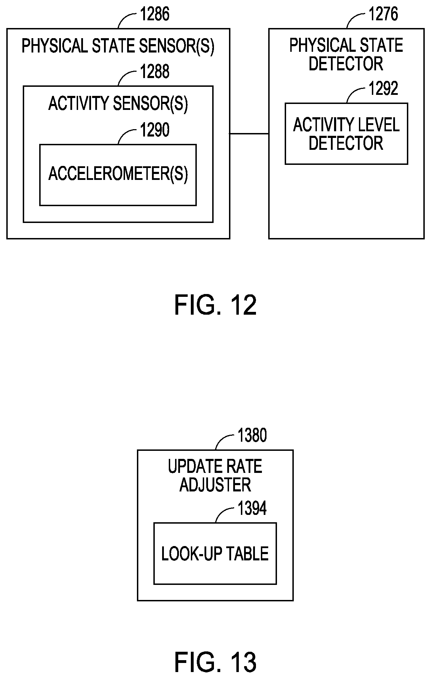

FIG. 12 illustrates an embodiment of one or more physical state sensors 1286 and a physical state detector 1276 for detecting a physical state of the patient. Physical state detector 1276 can represent an example of physical state detector 1076 and can detect a physical state of the patient using one or more physical signals sensed using physical state sensor(s) 1286. Physical state sensor(s) 1286 can be one or more sensors of sensors 960. Examples of the physical state to be detected by physical state detector 1276 include sleeping, cooking, sitting, exercising, showering, and walking.

In the illustrated embodiment, physical state sensor(s) includes one or more activity sensors 1288 that can detect one or more activity signals. Activity sensor(s) 1288 can include one or more accelerometers 1290 each producing an accelerometer signal being an activity signal. Physical state detector 1276 includes an activity level detector 1292 to detect an activity level using the sensed one or more activity signals. The activity level is a measure of a physical activity level of the patient. In various embodiments, the activity level can represent an overall physical activity level of the patient and can be determined using a single activity signal or a combination of activity signals. In various embodiments, activity level detector 1292 can produce discrete values of the activity level each corresponding to a range of the physical activity level of the patient. The discrete values can each correspond to a physical state of the patient (e.g., sleeping, cooking, sitting, exercising, showering, or walking), though whether the patient is actually in that physical state may need to be verified using additional information. In various embodiments, the activity level (e.g., representing the overall physical activity level of the patient) alone is sufficient for being used as a rate-adjusting parameter. Use of additional activity signals can require additional sensor(s) and/or increase computational activities and hence power consumption of implantable medical device 1104.

FIG. 13 illustrates an embodiment of an update rate adjuster 1380, which can represent an example of update rate adjuster 1080. In the illustrated embodiment, update rate adjuster 1080 includes a look-up table 1394 representing the relationship relating the sensing update rate to the one or more rate-adjusting parameters or the relationship relating each of the sensing update rate and the therapy update rate to the one or more rate-adjusting parameters. Update rate adjuster 1380 can adjust at least the sensing update rate based on the one or more rate-adjusting parameters using look-up table 1394. In various embodiments, update rate adjuster 1080 can adjust the sensing update rate and the therapy update rate using look-up table 1394.

The relationship represented by lookup table 1394 can be established using results from research and experiments. In various embodiments, the relationship can be established by balancing therapy and design considerations such as risk of inadequate therapy (e.g., excessive or insufficient neurostimulation), battery management considerations related to the sensing update rate and/or the therapy update rate, and available computational resources in implantable medical device 1104. In various embodiments, the relationship can be experimentally adjusted by updating look-up table 1394 for the patient based on related aspects of the performance of implantable medical device 1104 after being implanted in the patient. In various embodiments, the relationship reflects sensitivity of the sensing update rates and the therapy update rate to the change in the detected activity level (e.g., number of discrete values). In various embodiments in which the therapy includes delivery of neurostimulation pulses, the sensing update rate and the therapy update rate can be determined based on the pulse frequency (e.g., to allow the stimulation parameters to be adjusted for every pulse of neurostimulation pulses).

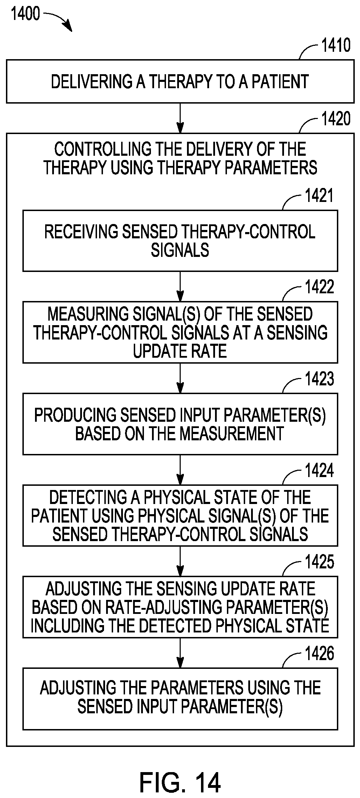

FIG. 14 illustrates an embodiment of a method 1400 for delivering a therapy to a patient. Method 1400 can be performed by system 1070 and any therapy device in which system 1070 is implemented, including but not limited to the therapy devices discussed in this document (e.g., stimulation device 104, stimulation device 204, IPG 404, IPG 504, IPG 604, implantable stimulator 704, therapy device 904, and implantable medical device 1104).

At 1410, the therapy is delivered to the patent. In various embodiments, the therapy is delivered from a therapy output device, such as a stimulation output circuit that can deliver neurostimulation.

At 1420, the delivery of the therapy is controlled using therapy parameters. In various embodiments, the delivery of the therapy is controlled using a therapy control circuit, such as a stimulation control circuitry that controls the delivery of neurostimulation using stimulation parameters. The stimulation output circuit and the stimulation control circuitry can be parts of an implantable neurostimulation device. Controlling the delivery of the therapy at 1420 can include steps 1421-1426 as illustrated in FIG. 14.

At 1421, sensed therapy-control signals are received from one or more sensors. The therapy-control signals include all the sensed signals that are used to control the delivery of the therapy, including input signals to a closed-loop control algorithm. In various embodiments, the sensed therapy-control signals include one or more electrophysiological signals sensed from the patient. When the therapy includes neurostimulation, the one or more electrophysiological signals can include one or more neural signals indicative of neural activities of the patient used for determining and adjusting the stimulation parameters. At 1422, one or more signals of the received sensed therapy-control signals are measured at a sensing update rate. At 1423, one or more sensed input parameters are produced based on the measurement.

At 1424, a physical state of the patient is detected using one or more physical signals of the received sensed therapy-control signals. An activity level of the patient can be detected as the physical state using one or more activity signals that can be sensed, for example, using one or more accelerometers. The activity level is a measure of a physical activity level of the patient. In various embodiments, the physical state is represented by the detected activity level only. In various other embodiments, one or more additional rate-adjusting parameters are used to determine the physical state of the patient, such as sleeping, cooking, sitting, exercising, showering, and walking. At 1425, the sensing update rate is adjusted based on one or more rate-adjusting parameters including the detected physical state. In various embodiments, the sensing update rate is adjusted using the detected physical state alone. In various other embodiments, the sensing update rate is adjusted using the detected physical state and one or more parameters quantitatively indicative of the energy and/or computational resource available in the therapy device. At 1426, the therapy parameters are adjusted using the one or more sensed input parameters. In various embodiments, the therapy parameters are adjusted at a therapy update rate that is also adjusted based on one or more rate-adjusting parameters including the detected physical state. In one embodiment, the sensing update rate and the therapy update rate are a common rate. In another embodiment, the sensing update rate is different from the therapy update rate. In various embodiments, the sensing update rate and the therapy update rate are each adjusted according to a relationship relating each of the sensing update rate and the therapy update rate to the one or more rate-adjusting parameters. The relationship can be determined based on research and experiments and stored for use as a look-up table. The relationship (e.g., the look-up table) can be adjusted for the patient experimentally as part of therapy adjustment performed periodically and/or as needed for the patient.

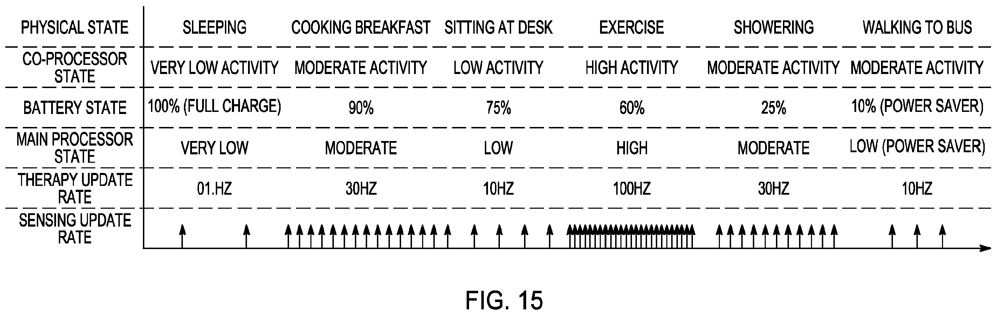

FIG. 15 illustrates an example of parameter update rates as a function of rate-adjusting parameters in implantable therapy device 1104 configured to be an implantable neurostimulator. The example uses multiple rate-adjusting parameters to adjust parameters update rates according to relationship between each of parameter update rates and the rate-adjusting parameters. The parameter update rates include the sensing update rate and the therapy update rate. The rate-adjusting parameters include the physical state of the patient, the co-processor state, the battery state, and the main processor state (as discussed above with respect to implantable therapy device 1104). The illustrated example shows the sensing update rate and the therapy update rate increase when the physical state corresponds to a higher activity level and decrease when the physical state corresponds to a lower activity level. Because the therapy parameters are adjusted in response to changes in the patient's needs and/or conditions, and such changes occur at a speed corresponding to the patient's physical state, using the physical state to determine the parameter update rates reduces unnecessary parameter updates (that includes little or no change in parameter values), thereby reducing the use of energy and computational resource. The co-processor state, the battery state, and the main processor state are also used for balancing the performance in controlling the delivery of the therapy between the energy and computational resources available and the speed of therapy adjustment in response to the changes in the changes in the patient's needs and/or conditions.

It is to be understood that the above detailed description is intended to be illustrative, and not restrictive. Other embodiments will be apparent to those of skill in the art upon reading and understanding the above description. The scope of the invention should, therefore, be determined with reference to the appended claims, along with the full scope of equivalents to which such claims are entitled.

## Claims

1. A system for delivering a therapy to a patient, the system comprising:
a therapy output device (912) configured to deliver the therapy to the patient; and
a therapy control circuit (914, 1014, 1114) configured to receive sensed therapy-control signals and to control the delivery of the therapy using the received sensed therapy-control signals, the therapy control circuit (914, 1014, 1114) including:
a therapy controller (1072) configured to control the delivery of the therapy using therapy parameters;
a therapy parameter adjuster (1074) configured to adjust the therapy parameters using one or more sensed input parameters at a therapy update rate;
a physical state detector (1076) configured to detect a physical state of the patient using one or more physical signals of the received sensed therapy-control signals; **characterised by**
a measurement system (1078) configured to measure one or more signals of the received sensed therapy-control signals to produce the one or more sensed input parameters based on the measurement at a sensing update rate; and
an update rate adjuster (1080) configured to adjust each of the therapy update rate and the sensing update rate based on one or more rate-adjusting parameters including the detected physical state.

2. The system according to claim 1, comprising an implantable medical device including the therapy output device (912) and the therapy control circuit (914, 1014, 1114).

3. The system according to any of the preceding claims, wherein the therapy output device (912) comprises a stimulation output circuit configured to deliver neurostimulation to the patient, and the therapy control circuit (914, 1014, 1114) comprises a stimulation control circuit configured to receive the sensed therapy-control signals and to control the delivery of the neurostimulation using the received sensed therapy-control signals.

4. The system according to any of the preceding claims, wherein the update rate adjuster (1080) is configured to adjust the sensing update rate based on the detected physical state and one or more parameters quantitatively indicative of at least one of energy or computational resource available to the therapy control circuit (914, 1014, 1114).

5. The system according to claim 4, wherein the therapy control circuit (914, 1014, 1114) comprises:
a main processor including the therapy controller (1072) and the therapy parameter adjuster (1074); and
a co-processor including the physical state detector (1076), the measurement system (1078), and the update rate adjuster (1080).

6. The system according to claim 5, further comprising a power source (748, 1148), and wherein the update rate adjuster (1080) is configured to adjust the sensing update rate based on the detected physical state and at least one of:
a battery state being a measure of the remaining energy in the power source (748, 1148);
a main processor state being a measure of a level of computational activities in the main processor; or
a co-processor state being a measure of a level of computational activities in the co-processor.

7. The system according to any of the preceding claims, wherein the sensing update rate and the therapy update rate comprise a common rate.

8. The system according to any of claims 1 to 6, wherein the sensing update rate is different from the therapy update rate.

9. The system according to any of the preceding claims, wherein the physical state detector (1076) comprises an activity level detector configured to detect an activity level as the physical state using one or more activity signals of the received sensed therapy-control signals, the activity level being a measure of a physical activity level of the patient, and wherein the update rate adjuster (1080) is configured to adjust the sensing update rate based on the one or more rate-adjusting parameters including the detected activity level.

10. The system according to claim 9, further comprising sensors (960) configured to be communicatively coupled to the therapy control circuit (914, 1014, 1114) and including one or more activity sensors configured to sense one or more activity signals.

11. The system according to claim 10, wherein the activity level detector is configured to detect the activity level using a single activity signal of the received sensed therapy-control signals.

12. The system according to claim 10, wherein the activity level detector is configured to detect the activity level using multiple activity signals of the received sensed therapy-control signals.

13. The system according to any of the preceding claims, wherein the update rate adjuster (1080) is configured to adjust the sensing update rate according to a relationship relating the sensing update rate to the one or more rate-adjusting parameters.

14. The system according to claim 13, wherein the update rate adjuster (1080) is configured to adjust the sensing update rate using a lookup table representative of the relationship.

## Patentansprüche

1. System zum Zuführen einer Therapie an einen Patienten, wobei das System aufweist:
eine Therapieausgabeeinrichtung (912), die dazu konfiguriert ist, die Therapie an den Patienten zuzuführen; und
einen Therapiesteuerschaltkreis (914, 1014, 1114), der dazu konfiguriert ist, erfasste Therapie-Steuersignale zu empfangen und die Zufuhr der Therapie unter Verwendung der empfangenen erfassten Therapie-Steuersignale zu steuern, wobei der Therapiesteuerschaltkreis (914, 1014, 1114) aufweist:
einen Therapiecontroller (1072), der dazu konfiguriert ist, die Zufuhr der Therapie unter Verwendung von Therapieparametern zu steuern;
einen Therapieparameteranpasser (1074), der dazu konfiguriert ist, die Therapieparameter unter Verwendung eines oder mehrerer erfasster Eingangsparameter mit einer Therapie-Aktualisierungsrate anzupassen;
einen Detektor für einen physischen Zustand (1076), der dazu konfiguriert ist, einen physischen Zustand des Patienten unter Verwendung eines oder mehrerer physischer Signale der empfangenen erfassten Therapie-Steuersignale zu detektieren;
**dadurch gekennzeichnet, dass**
ein Messsystem (1078) dazu konfiguriert ist, ein oder mehrere Signale der empfangenen erfassten Therapie-Steuersignale zu messen, um die eine oder mehreren erfassten Eingangsparameter basierend auf der Messung mit einer Erfassungs-Aktualisierungsrate zu erzeugen; und
ein Aktualisierungsratenanpasser (1080) dazu konfiguriert ist, jeweils die Therapie-Aktualisierungsrate und die Erfassungs-Aktualisierungsrate basierend auf einem oder mehreren ratenanpassenden Parametern, einschließlich des detektierten physischen Zustands, anzupassen.

2. System nach Anspruch 1, aufweisend ein implantierbares medizinisches Gerät, das die Therapieausgabeeinrichtung (912) und den Therapiesteuerschaltkreis (914, 1014, 1114) aufweist.

3. System nach einem der vorhergehenden Ansprüche, wobei die Therapieausgabeeinrichtung (912) einen Stimulationsausgabeschaltkreis aufweist, der dazu konfiguriert ist, Neurostimulation an den Patienten zuzuführen, und wobei der Therapiesteuerschaltkreis (914, 1014, 1114) einen Stimulationssteuerschaltkreis aufweist, der dazu konfiguriert ist, die erfassten Therapie-Steuersignale zu empfangen und die Zufuhr der Neurostimulation unter Verwendung der empfangenen erfassten Therapie-Steuersignale zu steuern.

4. System nach einem der vorhergehenden Ansprüche, wobei der Aktualisierungsratenanpasser (1080) dazu konfiguriert ist, die Erfassungs-Aktualisierungsrate basierend auf dem detektierten physischen Zustand und einem oder mehreren Parametern, die quantitativ eine Energie und/oder rechnerische Ressource angeben, die dem Therapiesteuerschaltkreis (914, 1014, 1114) zur Verfügung steht, anzupassen.

5. System nach Anspruch 4, wobei der Therapiesteuerschaltkreis (914, 1014, 1114) aufweist:
einen Hauptprozessor, der den Therapiecontroller (1072) und den Therapieparameteranpasser (1074) aufweist; und
einen Co-Prozessor, der den Detektor für einen physischen Zustand (1076), das Messsystem (1078) und den Aktualisierungsratenanpasser (1080) aufweist.

6. System nach Anspruch 5, ferner aufweisend eine Stromquelle (748, 1148), und wobei der Aktualisierungsratenanpasser (1080) dazu konfiguriert ist, die Erfassungs-Aktualisierungsrate basierend auf dem detektierten physischen Zustand und zumindest einem der folgenden anzupassen:
ein Batteriezustand als ein Maß für die verbleibende Energie in der Stromquelle (748, 1148);
ein Hauptprozessorzustand als ein Maß für ein Niveau rechnerischer Aktivitäten im Hauptprozessor; oder
ein Co-Prozessor-Zustand als ein Maß für ein Niveau rechnerischer Aktivitäten im Co-Prozessor.

7. System nach einem der vorhergehenden Ansprüche, wobei die Erfassungs-Aktualisierungsrate und die Therapie-Aktualisierungsrate eine gemeinsame Rate aufweisen.

8. System nach einem der Ansprüche 1 bis 6, wobei die Erfassungs-Aktualisierungsrate von der Therapie-Aktualisierungsrate verschieden ist.

9. System nach einem der vorhergehenden Ansprüche, wobei der Detektor für einen physischen Zustand (1076) einen Aktivitätsniveau-Detektor aufweist, der dazu konfiguriert ist, ein Aktivitätsniveau als den physischen Zustand unter Verwendung eines oder mehrerer Aktivitätssignale der empfangenen erfassten Therapie-Steuersignale zu detektieren, wobei das Aktivitätsniveau ein Maß für ein physisches Aktivitätsniveau des Patienten ist, und wobei der Aktualisierungsratenanpasser (1080) dazu konfiguriert ist, die Erfassungs-Aktualisierungsrate basierend auf den einen oder mehreren ratenanpassenden Parametern, einschließlich des detektierten Aktivitätsniveaus, anzupassen.

10. System nach Anspruch 9, ferner aufweisend Sensoren (960), die dazu konfiguriert sind, kommunikativ mit dem Therapiesteuerschaltkreis (914, 1014, 1114) gekoppelt zu werden, und die einen oder mehrere Aktivitätssensoren aufweisen, die dazu konfiguriert sind, ein oder mehrere Aktivitätssignale zu erfassen.

11. System nach Anspruch 10, wobei der Aktivitätsniveau-Detektor dazu konfiguriert ist, das Aktivitätsniveau unter Verwendung eines einzelnen Aktivitätssignals der empfangenen erfassten Therapie-Steuersignale zu detektieren.

12. System nach Anspruch 10, wobei der Aktivitätsniveau-Detektor dazu konfiguriert ist, das Aktivitätsniveau unter Verwendung mehrerer Aktivitätssignale der empfangenen erfassten Therapie-Steuersignale zu detektieren.

13. System nach einem der vorhergehenden Ansprüche, wobei der Aktualisierungsratenanpasser (1080) dazu konfiguriert ist, die Erfassungs-Aktualisierungsrate gemäß einer Beziehung anzupassen, die die Erfassungs-Aktualisierungsrate mit den einen oder mehreren ratenanpassenden Parametern in Beziehung setzt.

14. System nach Anspruch 13, wobei der Aktualisierungsratenanpasser (1080) dazu konfiguriert ist, die Erfassungs-Aktualisierungsrate unter Verwendung einer Nachschlagetabelle, die für die Beziehung repräsentativ ist, anzupassen.

## Revendications

1. Système d'administration d'une thérapie à un patient, le système comprenant :
un dispositif de sortie de thérapie (912) configuré pour administrer la thérapie au patient ; et
un circuit de commande de thérapie (914, 1014, 1114) configuré pour recevoir des signaux de commande de thérapie captés et pour commander l'administration de la thérapie à l'aide des signaux de commande de thérapie captés reçus, le circuit de commande de thérapie (914, 1014, 1114) incluant :
un équipement de commande de thérapie (1072) configuré pour commander l'administration de la thérapie à l'aide de paramètres de thérapie ;
un équipement d'ajustement de paramètre de thérapie (1074) configuré pour ajuster les paramètres de thérapie à l'aide d'un ou plusieurs paramètres d'entrée captés à une fréquence de mise à jour de thérapie ;
un détecteur d'état physique (1076) configuré pour détecter un état physique du patient à l'aide d'un ou plusieurs signaux physiques parmi les signaux de commande de thérapie captés reçus, **caractérisé par**
un système de mesure (1078) configuré pour mesurer un ou plusieurs signaux parmi les signaux de commande de thérapie captés reçus afin de produire les un ou plusieurs paramètres d'entrée captés sur la base de la mesure à une fréquence de mise à jour de détection ; et
un équipement d'ajustement de fréquence de mise à jour (1080) configuré pour ajuster chacune parmi la fréquence de mise à jour de thérapie et la fréquence de mise à jour de détection sur la base d'un ou plusieurs paramètres d'ajustement de fréquence incluant l'état physique détecté.

2. Système selon la revendication 1, comprenant un dispositif médical implantable incluant le dispositif de sortie de thérapie (912) et le circuit de commande de thérapie (914, 1014, 1114).

3. Système selon l'une quelconque des revendications précédentes, dans lequel le dispositif de sortie de thérapie (912) comprend un circuit de sortie de stimulation configuré pour administrer une neurostimulation au patient, et le circuit de commande de thérapie (914, 1014, 1114) comprend un circuit de commande de stimulation configuré pour recevoir les signaux de commande de thérapie captés et pour commander l'administration de la neurostimulation à l'aide des signaux de commande de thérapie captés reçus.

4. Système selon l'une quelconque des revendications précédentes, dans lequel l'équipement d'ajustement de fréquence de mise à jour (1080) est configuré pour ajuster la fréquence de mise à jour de détection sur la base de l'état physique détecté et d'un ou plusieurs paramètres indiquant quantitativement au moins l'une parmi une énergie ou une ressource de calcul disponibles pour le circuit de commande de thérapie (914, 1014, 1114).

5. Système selon la revendication 4, dans lequel le circuit de commande de thérapie (914, 1014, 1114) comprend :
un processeur principal incluant l'équipement de commande de thérapie (1072) et l'équipement d'ajustement de paramètre de thérapie (1074) ; et
un coprocesseur incluant le détecteur d'état physique (1076), le système de mesure (1078) et l'équipement d'ajustement de fréquence de mise à jour (1080).

6. Système selon la revendication 5, comprenant en outre une source de puissance (748, 1148), et dans lequel l'équipement d'ajustement de fréquence de mise à jour (1080) est configuré pour ajuster la fréquence de mise à jour de détection sur la base de l'état physique détecté et d'au moins l'un parmi :
un état de pile qui est une mesure de l'énergie restante dans la source de puissance (748, 1148) ;
un état de processeur principal qui est une mesure d'un niveau d'activités de calcul dans le processeur principal ; ou
un état de coprocesseur qui est une mesure d'un niveau d'activités de calcul dans le coprocesseur.

7. Système selon l'une quelconque des revendications précédentes, dans lequel la fréquence de mise à jour de détection et la fréquence de mise à jour de thérapie comprennent une fréquence commune.

8. Système selon l'une quelconque des revendications 1 à 6, dans lequel la fréquence de mise à jour de détection est différente de la fréquence de mise à jour de thérapie.

9. Système selon l'une quelconque des revendications précédentes, dans lequel le détecteur d'état physique (1076) comprend un détecteur de niveau d'activité configuré pour détecter un niveau d'activité en tant qu'état physique à l'aide d'un ou plusieurs signaux d'activité parmi les signaux de commande de thérapie captés reçus, le niveau d'activité étant une mesure d'un niveau d'activité physique du patient, et dans lequel l'équipement d'ajustement de fréquence de mise à jour (1080) est configuré pour ajuster la fréquence de mise à jour de détection sur la base des un ou plusieurs paramètres d'ajustement de fréquence incluant le niveau d'activité détecté.

10. Système selon la revendication 9, comprenant en outre des capteurs (960) configurés pour être couplés en communication avec le circuit de commande de thérapie (914, 1014, 1114) et incluant un ou plusieurs capteurs d'activité configurés pour détecter un ou plusieurs signaux d'activité.

11. Système selon la revendication 10, dans lequel le détecteur de niveau d'activité est configuré pour détecter le niveau d'activité à l'aide d'un unique signal d'activité parmi les signaux de commande de thérapie captés reçus.

12. Système selon la revendication 10, dans lequel le détecteur de niveau d'activité est configuré pour détecter le niveau d'activité à l'aide de multiples signaux d'activité parmi les signaux de commande de thérapie captés reçus.

13. Système selon l'une quelconque des revendications précédentes, dans lequel l'équipement d'ajustement de fréquence de mise à jour (1080) est configuré pour ajuster la fréquence de mise à jour de détection en fonction d'une relation reliant la fréquence de mise à jour de détection avec les un ou plusieurs paramètres d'ajustement de fréquence.

14. Système selon la revendication 13, dans lequel l'équipement d'ajustement de fréquence de mise à jour (1080) est configuré pour ajuster la fréquence de mise à jour de détection à l'aide d'une table de consultation représentative de la relation.
